# EUROPEAN PATENT SPECIFICATION

(11) **EP 3 347 029 B1**
(45) Date of publication and mention of the grant of the patent: **09.03.2022**
(21) Application number: 16844821.5
(22) Date of filing: 19.01.2016
(51) Int. Cl.: A61K 35/19

(54) **SYSTEM AND METHOD FOR PRODUCING BLOOD PLATELETS**
SYSTEM UND VERFAHREN ZUR HERSTELLUNG VON THROMBOZYTEN
SYSTÈME ET PROCÉDÉ POUR LA PRODUCTION DE PLAQUETTES SANGUINES

(30) Priority: 08.09.2015 US 201562215369 P
(43) Date of publication of application: 18.07.2018
(73) Proprietor: Brigham and Women's Hospital, Inc., Boston, MA 02115 (US)
(72) Inventor: THON, Jonathan, N., Dorchester, MA 02125 (US)
(74) Representative: Maiwald Patent- und Rechtsanwaltsgesellschaft mbH
(86) International application number: PCT/US2016/013855
(87) International publication number: WO 2017/044149

(56) References cited:
- WO-A1-2011/040889
- WO-A1-2014/100779
- WO-A1-2014/107240
- US-A1- 2005 224 351
- US-A1- 2012 014 933
- US-A1- 2014 147 880
- US-A1- 2014 186 414

## Description

### CROSS-REFENCE TO RELATED APPLICATIONS

### STATEMENT REGARDING FEDERALLY SPONSORED RESEARCH

This disclosure was made with government support under R00HL114719 awarded by the National Institutes of Health. The government has certain rights in the disclosure.

### BACKGROUND OF THE DISCLOSURE

The present disclosure generally relates to fluid systems, including microfluidic devices, systems that include such devices, and methods that use such devices and systems. More particularly, the present disclosure relates to devices, systems, and methods for generating biological products.

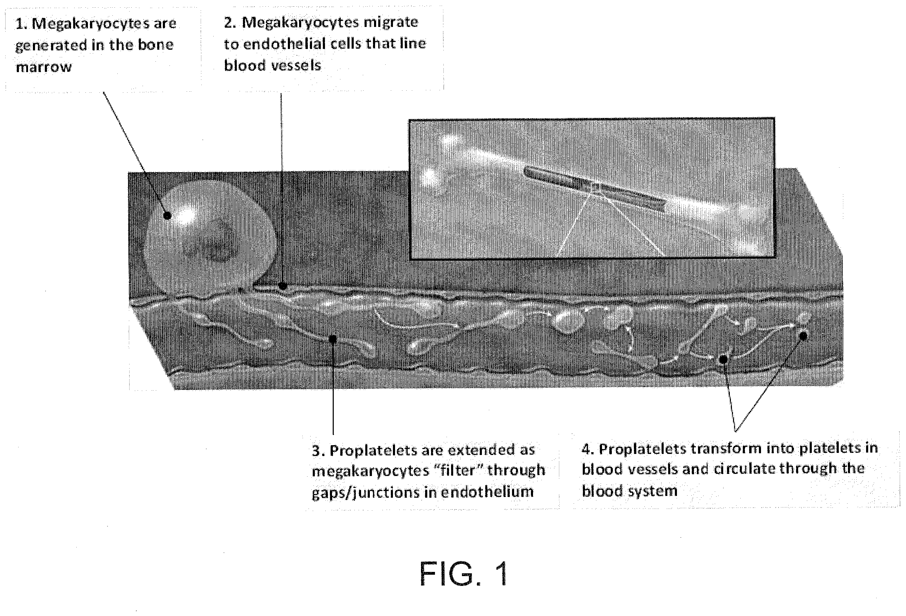

Blood platelets, or thrombocytes, are irregular, disc shaped cell fragments that circulate in the blood and are essential for hemostasis, angiogenesis, and innate immunity. In vivo, platelets are produced by cells, known as megakaryocytes. As illustrated in FIG. 1, megakaryocytes generated in the bone marrow migrate and contact endothelium cells that line blood vessels. There they extend long, branching cellular structures called proplatelets into the blood vessel space through gaps in the endothelium. Experiencing shear rates due to blood flow, proplatelets extend and release platelets into the circulation. In general, normal platelet counts range between 150,000 and 400,000 platelets per microliter of blood. However, when blood platelet numbers fall to low levels, a patient develops a condition known as thrombocytopenia and becomes at risk for death due to hemorrhage. Known causes for thrombocytopenia include malignancy and chemotherapy used to treat it, immune disorders, infection, and trauma.

Despite serious clinical concerns for deleterious immune system response, risk due to sepsis and viral contamination, treatment of thrombocytopenia generally involves using replacement platelets derived entirely from human donors. However, the process of obtaining platelets from transfusions is lengthy, costly, and often requires finding multiple matching donors. In addition, the usability of harvested platelets are limited due to a short shelf-life on account of bacterial testing and deterioration. Moreover, screening for viruses not known to exist is not possible. Combined with shortages created by increased demand and near-static pool of donors, it is becoming harder for health care professionals to provide adequate care for patients with thrombocytopenia, and other conditions related to low platelet counts. Alternatives to transfusion have included use of artificial platelet substitutes, these have thus far failed to replace physiological platelet products.

In some approaches, production of functional human platelets has been attempted using various cell culture techniques. Specifically, platelets have been produced in the laboratory using megakaryocytes obtained from various stem cells. Stem cells utilized have typically included embryonic stem cells, umbilical cord blood stem cells and induced pluripotent stem cells. Other stem cell sources have included stem cells found in bone marrow, fetal liver and peripheral blood. However, despite successful production of functional platelets in the laboratory, many limitations remain to use in a clinical setting.

For instance, only approximately 10% of human megakaryocytes have been shown to initiate proplatelets production using state-of-the art culture methods. This has resulted in yields of 10 to 100 platelets per CD34⁺ cord blood-derived or embryonic stem cell-derived megakaryocyte, which are themselves of limited availability. For example, the average single human umbilical cord blood unit can produce roughly 5·10⁶ CD34+ stem cells. This poses a significant bottleneck in *ex vivo* platelet production. In addition, cell cultures have been unable to recreate physiological microenvironments, providing limited individual control of extracellular matrix composition, bone marrow stiffness, endothelial cell contacts, and vascular shear rates. Moreover, cell cultures have been unsuccessful in synchronizing proplatelet production, resulting in non-uniform platelet release over a period of 6 to 8 days, which is on the order of platelet shelf-life.

Therefore, in light of the above, there remains a need for efficient ways to produce clinically relevant platelet yields that can meet growing clinical demands, and avoid the risks and costs associated with donor harvesting and storage.

US 2014/147880 and WO 2011/040889 disclose systems having first and second substrates with inlet and outlet channels, and permeable membranes forming microfluidic pathways between the channels.

### SUMMARY OF THE DISCLOSURE

The present disclosure overcomes the drawbacks of aforementioned technologies by providing a system and method capable of efficient and scalable production of platelets, and other biological products. Specifically, the disclosure describes various bioreactor embodiments that include a number of features and capabilities aimed at generating clinically and commercially relevant biological products. In some aspects, the system and method described herein may be used to generate high platelet yields usable for platelet infusion. As such, many significant drawbacks of present replacement therapies can be overcome, since these predominantly rely on transfusions from human donors.

As will be described, in some aspects, provided bioreactor embodiments can be configured to recreate physiological conditions and processes associated with platelet production in the human body. Specifically, provided bioreactor embodiments can be configured for selective functionalization using various materials and substances that can facilitate platelet production. Also, by including capabilities for uniform biological material trapping and controllable shear stresses, efficient production of platelets, and other biological products, can be achieved using the bioreactor embodiments described. In some designs, provided bioreactor embodiments are configured for rapid assembly and disassembly, and adaptable to thermoplastic molding and other large scale manufacturing processes.

The invention provides a system for generating biological products, the system comprising:a first substrate having formed therein a number of inlet channels extending substantially along a longitudinal direction;a second substrate having formed therein a number of outlet channels corresponding to the number of inlet channels and extending substantially along the longitudinal direction, the second substrate configured to releasably engage the first substrate; and arranged between adjacent surfaces of the substrates, a permeable membrane forming microfluidic pathways between the respective inlet and outlet channels and configured to selectively capture biological source material capable of generating biological products,wherein in a cross-sectional view of the system that includes the longitudinal direction and a height direction at least one of a given inlet channel or a given outlet channel corresponding to the given inlet channel is tapered relative to the longitudinal direction at a first angle to control a pressure differential profile regulating perfusion of a fluid medium through the permeable membrane, and wherein the tapering of the at least one of the given inlet channel or the given outlet channel relative to the longitudinal direction adjusts a shear stress in the channels.

In accordance with another aspect of the disclosure, which is not claimed, a method for generating biological products is provided. The method includes seeding a bioreactor assembly with biological source material capable of generating desired biological products, the bioreactor assembly a first substrate having formed therein a plurality of inlet channel extending substantially along a longitudinal direction, and a second substrate, configured to releasably engage the first substrate, and having formed therein a plurality of outlet channel corresponding to the plurality of inlet channel and extending substantially along the longitudinal direction, wherein at least one channel is tapered transversally to control a pressure differential profile therein. The bioreactor assembly also includes, a permeable membrane, arranged between the substrates, forming microfluidic pathways between respective inlet and outlet channels and configured to selectively capture biological source material. The method also includes introducing fluid media into the bioreactor assembly at predetermined flow rates to generate the desired biological products, and harvesting the desired biological products from the bioreactor assembly.

The foregoing and other aspects and advantages of the disclosure will appear from the following description. In the description, reference is made to the accompanying drawings which form a part hereof, and in which there is shown by way of illustration a preferred embodiment of the disclosure. Such embodiment does not necessarily represent the full scope of the disclosure, however, and reference is made therefore to the claims and herein for interpreting the scope of the disclosure.

### BRIEF DESCRIPTION OF THE DRAWINGS

The present disclosure will hereafter be described with reference to the accompanying drawings, wherein like reference numerals denote like elements.
FIG. 1 is an illustration showing *in vivo* platelet production in bone marrow.
FIG. 2 is a schematic diagram of a system for producing biological products, in accordance with aspects of the present disclosure.
FIG. 3 is an illustration showing an embodiment of a bioreactor, in accordance with aspects of the present disclosure.
FIG. 4A is a perspective view showing one embodiment of a bioreactor assembly including the bioreactor of FIG. 3.
FIG. 4B is a cross-sectional view of the embodiment shown in FIG. 4A.
FIG. 4C is another cross-section view of the embodiment shown in FIG. 4A.
FIG. 5A shows an example system, in accordance with aspects of the present disclosure.
FIG. 5B shows the bioreactor included in the system of FIG. 5A.
FIG. 6 shows an embodiment of a bioreactor, in accordance with aspects of the present disclosure.
FIG. 7A shows an embodiment of a bioreactor assembly that is disassembled, in accordance with aspects of the present disclosure.
FIG. 7B shows the bioreactor assembly of FIG. 7A assembled.
FIG. 8 shows another embodiment of a system, in accordance with aspects of the present disclosure.
FIG. 9A shows yet another embodiment of a bioreactor, in accordance with aspects of the present disclosure.
FIG. 9B shows yet another embodiment of a bioreactor, in accordance with aspects of the present disclosure.
FIG. 10 is a schematic showing a system, in accordance with aspects of the present disclosure.
FIG. 11 shows yet another embodiment of a bioreactor, in accordance with aspects of the present disclosure.
FIG. 12 shows yet another embodiment of a bioreactor, in accordance with aspects of the present disclosure.
FIG. 13 is a flowchart setting forth steps of a process, in accordance with aspects of the present disclosure.
FIG. 14A is a graph showing megakaryocytes produced using a static culture.
FIG. 14B is a graph showing proplatelets and platelets produced using a static culture.
FIG. 14C is a graph showing megakaryocytes produced in accordance with aspects of the present disclosure.
FIG. 14D is a graph showing proplatelets and platelets produced in accordance with aspects of the present disclosure.
FIG. 15 is a graph showing a timeline of platelet production, in accordance with aspects of the present disclosure.
FIG. 16 shows yet another embodiment of a bioreactor, in accordance with aspects of the present disclosure.

### DETAILED DESCRIPTION OF THE DISCLOSURE

The present disclosure provides systems and methods capable of efficient and scalable production of platelets, and other biological products.

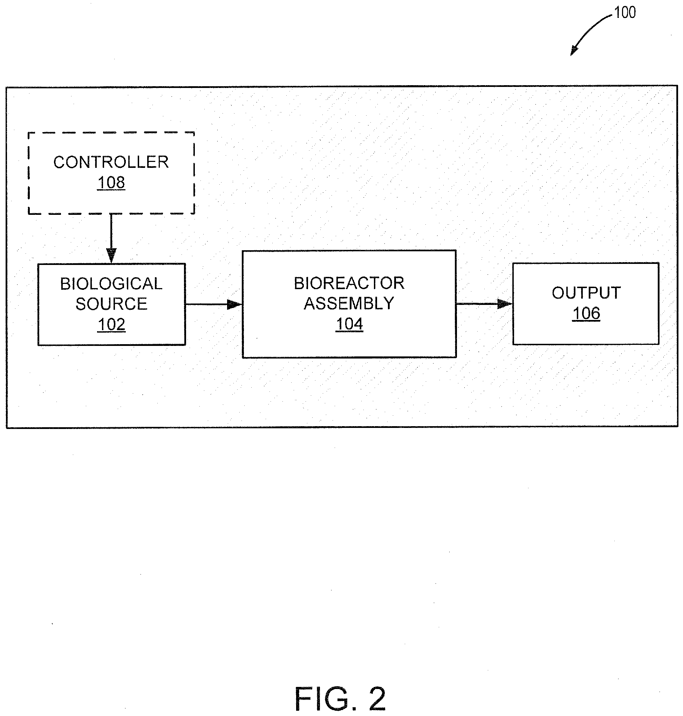

Turning now to FIG. 2, a schematic diagram of an example system 100 for producing platelets, and other biological products, is shown. In general, the system 100 includes a biological source 102, a bioreactor assembly 104, and an output 106, where the biological source 102 and output 106 are connectable to various inputs and outputs of the assembly 104, respectively.

Specifically, the biological source 102 may be configured with various capabilities for introducing into the assembly 104 different biological source materials, substances, gas, or fluid media, to efficiently produce desirable biological products, such as platelets. For instance, the biological source 102 may include one or more pumps for delivering or sustaining fluid media in the bioreactor assembly 104. Examples include microfluidic pumps, syringe pumps, peristaltic pumps, and the like.

As shown in FIG. 2, in some implementations, the system 100 may also include a controller 108 for controlling the biological source 102. Specifically, the controller 108 may be a programmable device or system configured to control the operation of the bioreactor assembly 104, including the timings, amounts, and types of biological source material, substances, fluid media or gas introduced therein. In some aspects, the controller 108 may be configured to selectively functionalize and/or operate the assembly 104 to recreate physiological conditions and processes associated with platelet production in the human body. For example, the controller 108 may be programmed to deliver a selected number of megakaryocytes to the assembly 104. In addition, the controller 108 may control fluid flow rates or fluid pressures in the bioreactor assembly 104 to facilitate proplatelet extension and platelet production. For instance, the controller 108 may establish flow rates up to 150,000 microliters/hr in various channels configured in the bioreactor assembly 104.

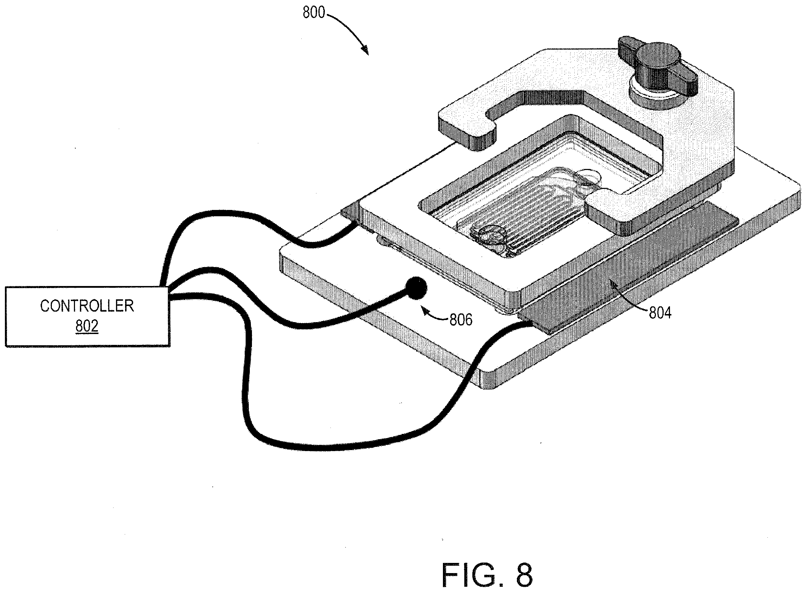

Although the controller 108 is shown in FIG. 2 as separate from the biological source 102, it may be appreciated that these may be combined into a single unit. For instance, the biological source 102 and controller 106 may be embodied in a programmable microfluidic pump or injection system. In addition, in some implementations, the controller 108 and/or biological source 102 may also include, communicate with, or received feedback from systems or hardware (not shown in FIG. 2) that can regulate the temperature, light exposure, vibration, and other conditions of the bioreactor assembly 104. By way of example, FIG. 8 shows a bioreactor system 800 that includes a controller 802 connected to heaters 804 and a thermocouple 806 for monitoring and controlling temperature. It may be appreciated that the configuration shown in FIG. 8 is non-limiting, and any number of heaters, and heater arrangements may be possible.

Referring again to FIG. 2, in general, the output 106 is configured to receive fluid media containing various biological products generated in the bioreactor assembly 104. However, in some implementations, as will be described, such effluent may be redirected or circulated back into the bioreactor assembly 104. In this manner, less fluid volume may be utilized, and the biological products generated can be more concentrated. In some aspects, the output 106 may also include capabilities for collecting, storing and/or further processing received fluid media.

It may be appreciated that the above-described system 100 has a broad range of functionality, and need not be limited to replicating physiological conditions or processes, nor producing platelets. That is, the system 100 may be used to generate a wide variety of biological products. For instance, the system 100 may be used to separate, break up or dissolve various biological source materials or substances, such as megakaryocytes and other cells, and collect their product or content. Specifically, by controlling fluid flow and pressures, as well as other conditions, various contents of captured biological source materials may be released and subsequently harvested. In some aspects, the system 100 may also be utilized to differentiate and/or culture various cells, biological substances or materials, such as megakaryoctytes, for obtaining biological source material needed to generate desirable biological products. Example biological products include growth factors, and other components found in cells. Produced biological products, in accordance with the present disclosure, in addition to clinical use, may find use in a variety of applications including as components of cell culture medias and cosmaceuticals, such as cosmetics, shampoos, skin additives, creams, or cleaners, and so forth.

Various embodiments of the above system 100 will now be described. It may be appreciated that these are non-limiting examples, and indeed various modifications or combinations are possible and considered by one of ordinary skill in the art to be within the intended scope of the present application.

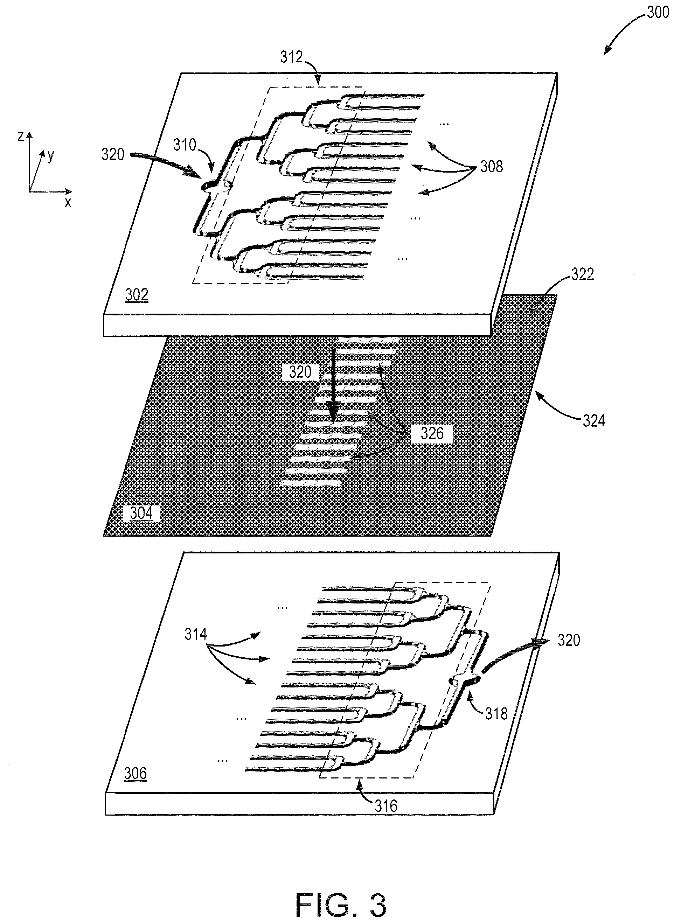

Referring now to FIG. 3, an non-limiting microfluidic bioreactor 300, in accordance with aspects of the present disclosure, is shown. In general, the bioreactor 300 includes a first substrate 302, a second substrate 306, and a permeable membrane 304 arranged therebetween.

In particular, the first substrate 302 can include a number of inlet channels 308, or inlet chambers, formed therein. As shown in FIG. 3, in some embodiments, the channels can be arranged generally parallel to one another and extending substantially along a longitudinal direction (for example, the x direction). However it may be appreciated that any channel arrangement capable of achieving platelet and other biological product production, as described in the present disclosure, may be possible. Also, the inlet channels 308 may be connected to an inlet port 310 by way of an inlet manifold 312 formed in the first substrate 302, where the inlet manifold 312 is configured to provide similar or comparable fluid pathways for fluid media traversing therethrough. Such configuration may be advantageous for distributing various biological substances or materials uniformly across the bioreactor 300, or can be manipulated to distribute various biological substances or materials selectively or differentially within different fluid pathways. For instance, a concentration of platelet-producing megakaryocyte cells dispersed in a fluid medium can be introduced in one infusion step into the inlet port 310 to achieve a similar density or spatial distribution across the permeable membrane 304. In alternative configurations, each inlet channel 308 may include different sized or separate inlet ports.

The second substrate 306 can include a plurality of outlet channels 310, or outlet chambers, each corresponding to a respective inlet channel, and also extending substantially parallel along the longitudinal direction. Similarly, the second substrate 304 may also include an outlet manifold 316 formed therein, the outlet manifold 316 connected to the outlet channels 314 and configured to direct fluid media, including various biological products, substances or materials, to an output via an outlet port 318.

By way of example, the substrates described above may have lateral dimensions in a range between 10 mm and 100 mm, and a thickness in a range between 1 and 10 mm, although other dimensions may also be possible. Also, a longitudinal dimension of the inlet channels 308 and/or outlet channels 314 be in the range of 1000 to 30,000 micrometers or, more particularly, in the range of 1000 to 3000 micrometers, while at least one transverse dimension may be in the range of 100 to 3,000 micrometers or, more particularly, in the range of 100 to 300 micrometers. Other dimensions are also possible. As will be described, in some embodiments, the inlet channels 308 or outlet channels 314 may also be tapered transversally either entirely or over a portion of the longitudinal dimension to control shear rates or pressure differentials between the channels, over an active contact area, regulating perfusion through the permeable membrane 304.

Various implementations of the bioreactor 300 are possible depending upon specific uses or applications. In particular, in some embodiments, the inlet channels 308 and outlet channels 314, along with other microfluidic elements of bioreactor 300, may be longitudinally (for example, along the x-direction shown in FIG. 3) and transversally (y- and z-direction) shaped and dimensioned to reproduce physiological conditions, such as those found in bone marrow and blood vessels. For instance, channel shapes and dimensions may be selected to achieve physiological flow rates, shear rates, fluid pressures or pressure differentials similar to those associated with *in vivo* platelet production, as described with reference to FIG. 1. In addition, configurations of the bioreactor 300 may be chosen to allow cooperation with other instrumentation, such as microscopes or cameras. For instance, the bioreactor 300 may be configured to adhere to standard microplate dimensions.

In some configurations of the bioreactor 300, the inlet channels 308 and outlet channels 314 terminate in their respective substrates to create a single fluid conduit 320 from the inlet port 310 to the outlet port 318, as shown in FIG. 3. That is to say, fluid medium introduced into the inlet port 310 is necessarily extracted from the outlet port 318. However, it may appreciated that additional inlet and outlet ports may also be possible with the bioreactor 300. For example, the first substrate 302 may also include one or more outputs connected to the inlet channels 308. Similarly, the second substrate 306 may also include one or more inputs connected to the outlet channels 314. In this manner, multiple fluid pathways can be possible, which would allow for selectively preparing various portions of the bioreactor 300 independently.

Although not shown in FIG. 3, the bioreactor 300 may also include a number of microfluidic filtration and resistive elements, connected to the channels and arranged at various points along the various fluid pathways extending between the inlet port 310 and outlet port 318. For instance, one or more filtration elements may be placed proximate to the inlet port 310 to capture contaminants or undesirable substances or materials from an inputted fluid medium. In addition, one or more resistive elements may also be included to control flow forces or damp fluctuations in flow rates. In addition to resistive and filtration elements, elements may also be included. For example, air bubbles traps may be configured with one or more inputs to eliminate any air bubbles from entering the bioreactor 300.

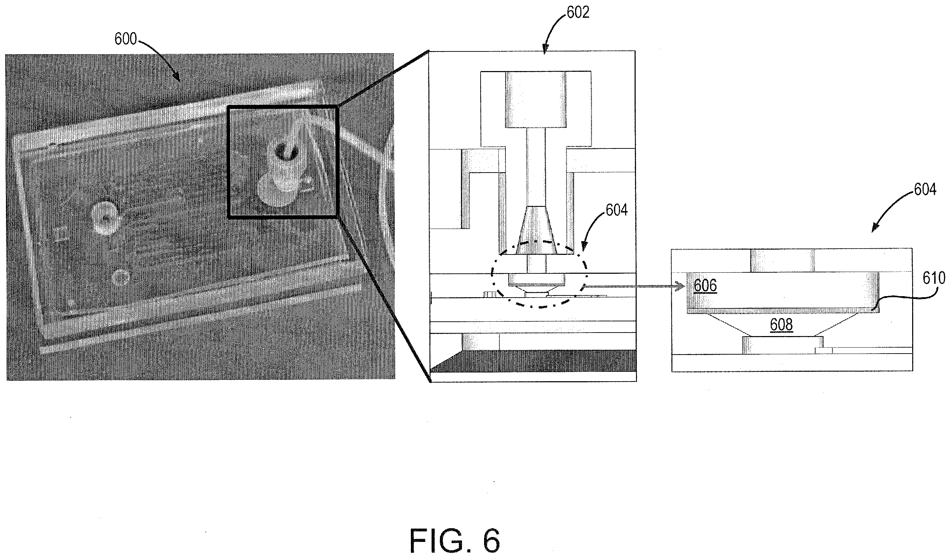

By way of example, FIG. 6 illustrates a microfluidic connector 602 coupled to an input 604 of an example bioreactor 600, in accordance with the above descriptions. As shown, the input 604 includes an expansion region 606 and a conical region 608 separated by a mesh 610. For example, the mesh 610 may have a size of approximately 140 micrometers, although other values may be possible. As configured, the input 604 is capable of preventing air bubbles from entering the bioreactor 600.

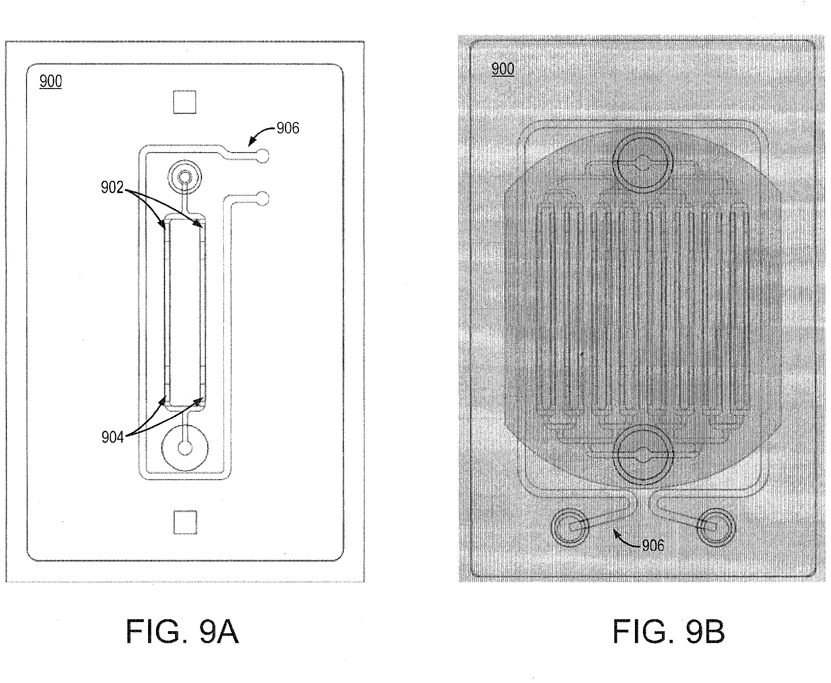

Referring again to the bioreactor 300 of FIG. 3, each substrate is shown to include 16 inlet channels 312 and 16 outlet channels 314. It may be readily appreciated that more or fewer channels or chambers may be possible. For example, as illustrated in the bioreactor 900 shown in FIG. 9A, two inlet channels 902 and two outlet channels 904 may be utilized. In addition to inlet and outlet channels, additional conduits may also be formed in the substrates of the bioreactor 300 of FIG. 3. For instance, as shown in FIG. 9A and 9B, a perfusion channel 906 may also be included in the bioreactor 900. Specifically, the perfusion channel 906 would allow the flow of gas, which can subsequently perfuse through the substrate materials and into the bioreactor 900 inlet/outlet channels. For example, a 5% CO₂ gas mixture may be perfused into the channels.

In general, the permeable membrane 304 of FIG. 3 can include any rigid or flexible layer, film, mesh or material structure configured to connect corresponding inlet channel 308 and outlet channel 314 via microfluidic pathways formed therein. In one embodiment, microfluidic pathways in the permeable membrane 304 may be formed using pores, gaps or microchannels, distributed with any density, either periodically or aperiodically, about permeable membrane 304. In another embodiment, the permeable membrane 304 can include a three-dimensional structure formed using interwoven micro- or nano-fibers arranged to allow fluid therethrough. Although shown in FIG. 3 as rectangular in shape, it may be appreciated that the permeable membrane 304 may have any shapes, including circular shapes, oval shapes, and so forth. In accordance with aspects of the disclosure, the permeable membrane 304 may be configured to selectively capture specific biological source materials or substances to produce desired biological products. For instance, the permeable membrane 304 may be configured to selectively capture platelet-producing cells and allow proplatelet extensions therethrough.

By way of example, the permeable membrane 304 may include longitudinal and transverse dimensions in a range between 1 and 100 millimeters, and have a thickness in a range between 0.1 to 20 micrometers, although other dimensions are possible. Also, the permeable membrane 304 may include pores, gaps or microchannels sized in a range approximately between 3 micrometers and 10 micrometers, and more specifically approximately between 5 and 8 micrometers. In some aspects, pore, gap or microchannel size, number, and density may depend on a number of factors, including desired biological products and product yields, as well as flow impedances, shear rates, pressure differentials, fluid flow rates, and other operational parameters.

As appreciated from FIG. 3, the inlet channels 308 and outlet channels 314 overlap to define an active contact area in the permeable membrane 304. For example, an active contact area 326 may be in a range between 1 mm² to 20 mm², although other values are possible, depending upon the dimensions and number of channels utilized. In some implementations, the active contact area along with permeable membrane 304 characteristics may be optimized to obtain a desired biological product yield. For example, a permeable membrane 304 with 47 mm diameter, 5% active contact area, and pore density of 1·10⁵ pores/cm² could provide 200,00 potential sites for generating a desired biological product yield, such as a desired platelet yield. In some applications, the active contact area may be configured to trap at least approximately 1·10⁴ megakaryocytes.

The bioreactor 300 may be manufactured using any combination of biocompatible materials, inert materials, as well as materials that can support pressurized gas and fluid flow, or gas diffusion, and provide structural support. In some aspects, materials utilized in the bioreactor 300 may be compatible with specific manufacturing processes, such as injection molding. In addition, materials utilized may optically clear to allow visualization of fluid media, and other substances, present or flowing in various portions of the bioreactor 300.

By way of example, the first substrate 302, or second substrate 306, or both, or portions thereof, may be manufactured using cell-inert silicon-based organic polymer materials, such as polydimethylsiloxane ("PDMS"), thermoplastic materials, such as zeonor cyclo olefin polymer ("COP"), glass, acrylics, and so forth. On the other hand, the permeable membrane 304 may be manufactured using PDMS, thermoplastics, silk, hydrogels, extracellular matrix proteins, polycarbonate materials, polyesthersulfone materials, polyvinyl chloride materials, polyethyleneterephthalat materials, and other synthetic or organic materials.

In accordance with aspects of the present disclosure, the bioreactor 300 may be selectively functionalized using various biological substances and materials. Specifically, the bioreactor 300 may be selectively functionalized by way of fluid media, containing desired biological substances and materials, being introduced therein. Alternatively, or additionally, the bioreactor 300, or components thereof, may be functionalized using various preparation or manufacturing processes. For example, the permeable membrane 304 may be pre-prepared with platelet-producing cells prior to assembly of the bioreactor 300. In some aspects, the bioreactor 300 may be utilized to differentiate and/or culture megakaryocytes, as well as other cells, biological substances or materials.

As described, in some aspects, the bioreactor 300 may be advantageously functionalized to replicate *in vivo* physiological conditions in order to produce platelets, or other biological products. For instance, in one application, a top surface 322 of the permeable membrane 304 may be selectively coated with extracellular matrix proteins, for example, while a bottom surface 322 can be left without, or can be coated with different proteins or substances. This can be achieved, for instance, by infusing a first fluid medium containing extracellular matrix proteins, using inputs and outputs in the first substrate 302. At substantially the same time, a second fluid medium flow can be maintained in the second substrate 306 using respective inputs and outputs, where the second fluid medium would either contain no proteins, or different proteins or substances. Preferably, flow rates of the first and second fluid media would be configured such that little to no fluid mixing would occur. Such selective functionalization would ensure that introduced platelet-producing cells, for example, coming to rest on the top surface 322 would contact extracellular matrix proteins, while proplatelets extended through the permeable membrane 304, and platelets released therefrom, would not contact extracellular matrix proteins, or would contact different proteins or biological substances.

Non-limiting examples of biological substances and materials for functionalizing the bioreactor 300 may include human and non-human cells, such as megakaryocytes, endothelial cells, bone marrow cells, osteoblasts, fibroblasts, stem cells, blood cells, mesenchymal cells, lung cells and cells comprising basement membranes. Other examples can include small molecules, such as CCL5, CXCL12, CXCL10, SDF-1, FGF-4, S1PR1, RGDS, Methylcellulose. Yet other examples can include, extracellular matrix proteins, such as bovine serum albumin, collagen type I, collagen type IV, fibrinectin, fibrinogen, laminin, vitronectin. In particular, to replicate three-dimensional extracellular matrix organization and physiological bone marrow stiffness, cells may be infused in a hydrogel solution, which may subsequently be polymerized. The hydrogel solution may include, but is not limited to alginate, matrigel, agarose, collagen gel, fibrin/fibrinogen gel.

In some aspects, various portions of the bioreactor 300 may be configured to allow for assembly and disassembly. Specifically, as shown in FIG. 3, the first substrate 302, permeable membrane 304, and second substrate 306 may be configured to be removably coupled to one another. When engaged using fasters, clips, or other releasable locking mechanisms, for example, a hermetic seal can then be formed between various surfaces of the substrates and permeable membrane 304 to reinstate fluid pathway integrity between the input 310 and output 318. This capability can facilitate preparation, as described above, as well as cleaning for repeated use. In addition, disassembly allows for quick exchange of various components, for repurposing or rapid prototyping. For instance, a permeable membrane 304 having different pore sizes, or different preparations, may be readily swapped.

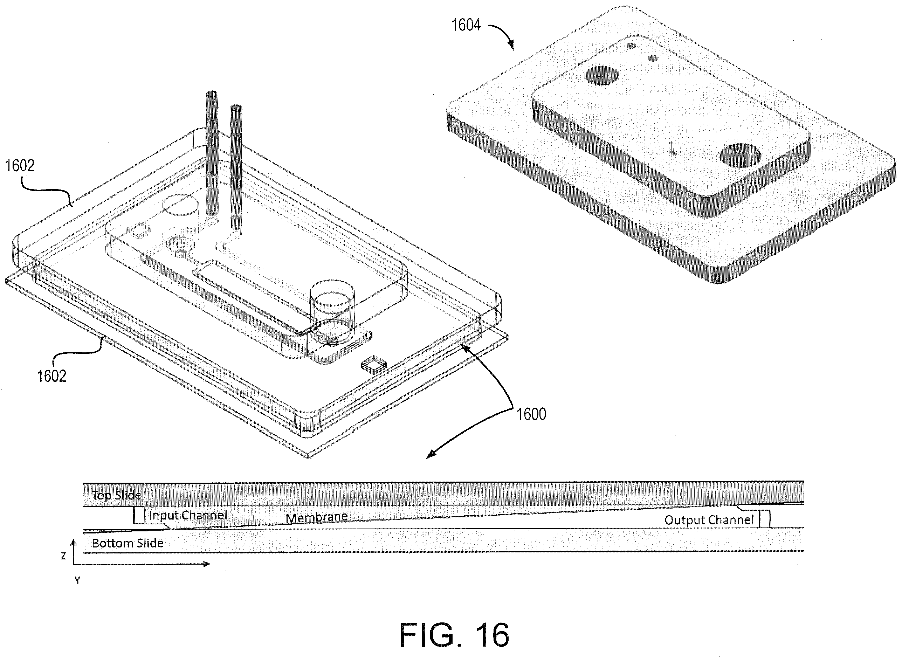

Alternatively, the bioreactor 300 may be manufactured as a single device, for sample using an injection molding technique, where the permeable membrane 304 would be molded into the substrates. Such implementations may be advantageously integrated into large scale manufacturing techniques. By way of example, FIG. 16 shows a bioreactor 1600 generated using a molding technique. The bioreactor 1600 may be formed using PDMS, thermoplastic, such as copolymer ("COP"), cyclic olefin copolymer ("COC"), polymethylmethacrylate ("PMMA"), polycarbonate ("PC"), and other materials. As shown in FIG. 16, in some embodiments, the bioreactor 1600 may be include a glass substrate 1602 either on the top, or the bottom, of the bioreactor 1600 or both. Alternatively, the bioreactor 1600 may include an acrylic top substrate 1604, alone or in combination with a glass or acrylic substrate. These substrates could be used provide structural support to the bioreactor 1600.

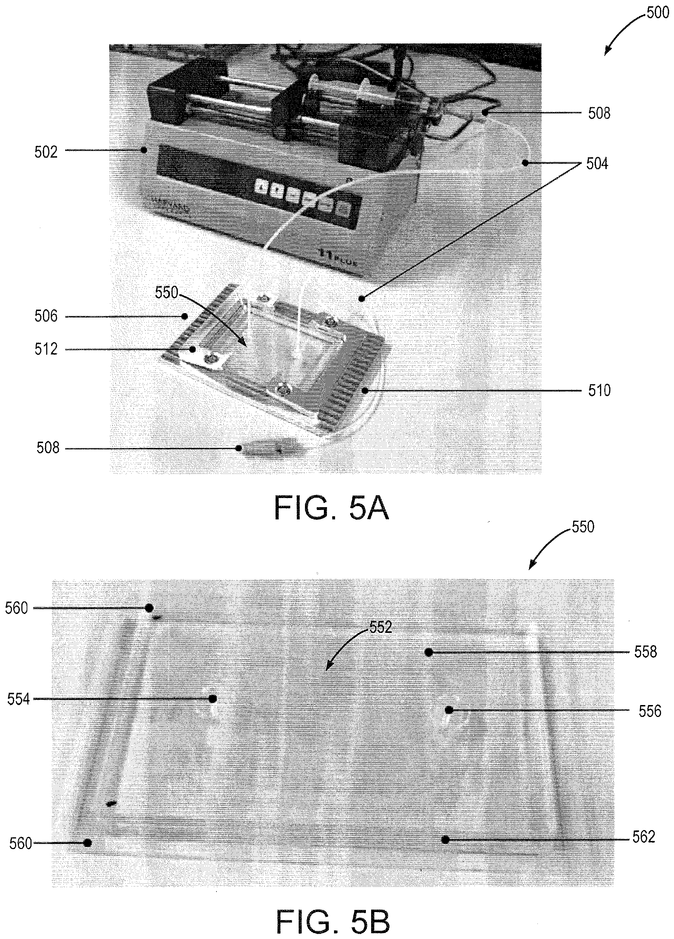

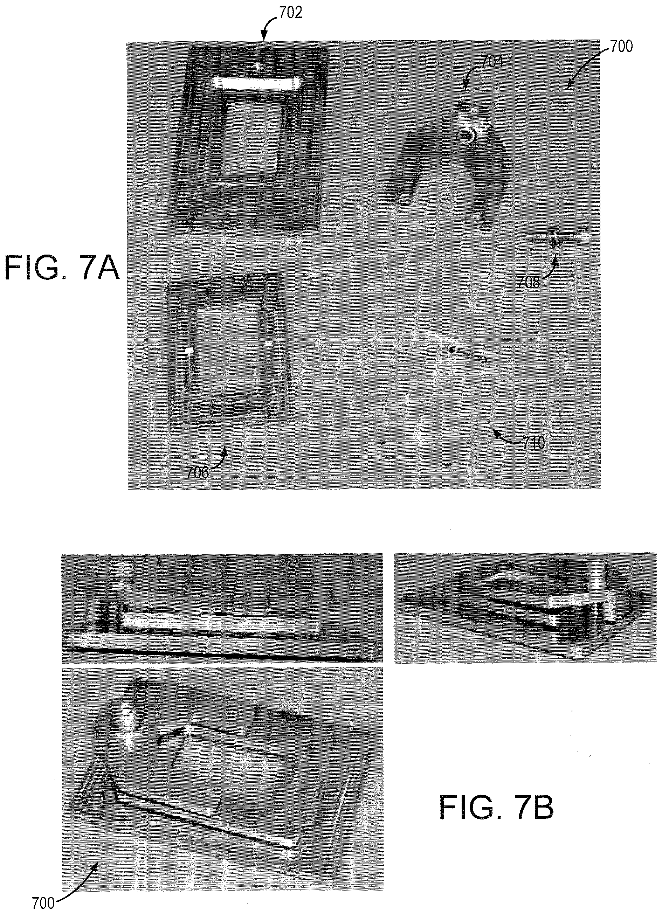

By way of example, FIGs. 7A-B show an example bioreactor assembly 700 both disassembled and assembled, respectively. As shown in FIG. 7A, the bioreactor assembly 700 in general includes a base plate 702, a clamp arm 704, a top clamp frame 706, fastener 708, and bioreactor 710. When assembled, as shown in FIG. 7B, a compression is provided to the bioreactor 710 bringing respective substrates of the bioreactor 710 to form a hermetic seal therebetween. In some designs, the bioreactor assembly 700 may incorporate hard stops with springs to be able to apply an even pressure across top and bottom surfaces of the bioreactor 710 to prevent leaks and avoid over-compression. Other fastening mechanisms or variations thereof are also possible, for example, as shown in FIG. 5A, or FIG. 8.

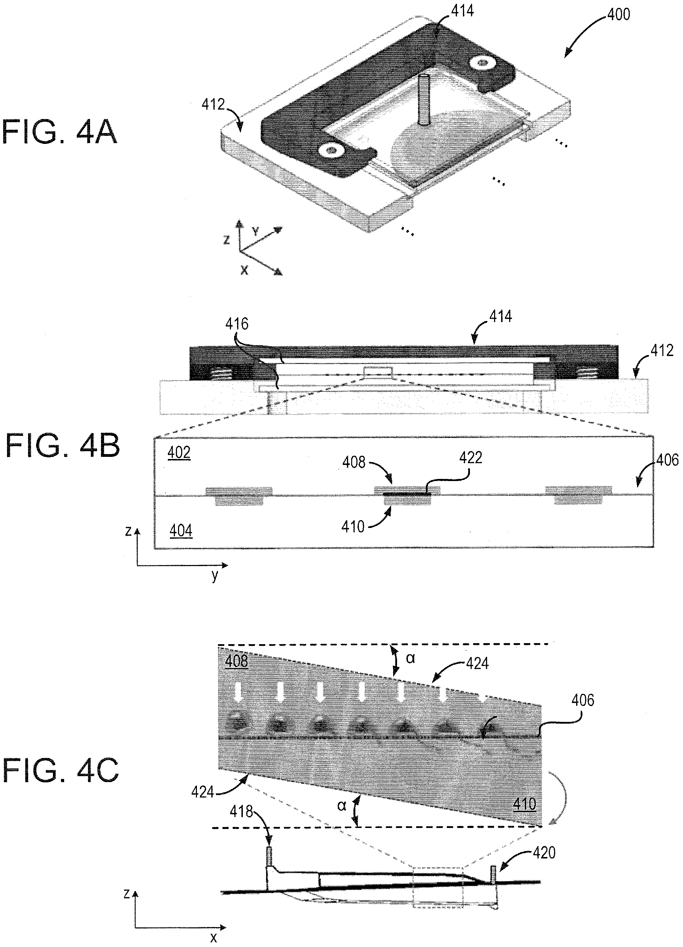

Referring now to FIGs. 4A-4C, a non-limiting embodiment of a microfluidic bioreactor assembly 400, in accordance with aspects of the present disclosure, is illustrated. Referring specifically to the cross-sectional view of FIG. 4B, the bioreactor assembly 400 can include a top substrate 402, a bottom substrate 404, and a membrane 406 therebetween that connects a number of corresponding inlet channels 408 and outlet channels 410 formed in respective substrates. As described, the membrane 406 is configured to form fluid pathways between the substrates, for instance, via pores included therein. As shown in the perspective view of FIG. 4A, the bioreactor assembly 400 can also include a base portion 412 and top portion 414 configured to fasten the membrane 406 and substrates together, for example using screws. For simplicity, FIG. 4A shows only a portion of the bioreactor assembly 400. When fastened, the base portion 412 and top portion 414 bring the substrates into an aligned engagement (for example using guides, grooves, or holes) capable of forming a hermetic seal therebetween in order to allow fluid or gas flow, or both, in the channels without leaks. Conversely, when unfastened, various portions of the bioreactor assembly 400 can be individually prepared, exchanged, cleaned and reused.

As described, in some configurations, the bioreactor assembly 400 may be configured to allow visualization during operation. Referring specifically to FIG. 4B, each substrate may have a glass layer 416 arranged proximate thereto, forming a stack that includes glass/substrate/membrane/substrate/glass. In alternative configurations, the glass layers may be excluded, with the substrates and/or membrane 406 configured to have appropriate structural integrity and desired material characteristics, such as transparency appropriate for allowing visualization of fluid media therethrough. As may be appreciated, the specific configurations of the bioreactor assembly 400 and portions thereof, as well as manner of assembly, may be modified or adapted to the specifics of the particular application.

The inlet channels 408 and outlet channels 410 of the bioreactor assembly 400 need not have equal dimensions. That is, as shown in FIG. 4B, the inlet channels 408 can be larger (or smaller) in at least one transverse (or longitudinal) dimension compared to the outlet channels 410. By way of example, the inlet channels may have a first transverse dimension, or channel height of approximately 0.1 mm, a second transverse dimension, or channel width of approximately 0.7 mm, while the outlet channels may have a channel height of approximately 0.1 mm and a channel width of approximately 0.5 mm. The channels may have a longitudinal dimension or length of approximately 25 mm. Various other dimensions may be possible. In some aspects, dimensions, including channel depths and widths, may be configured such that megakaryocytes experience desired shear stress in the center of the channels, and avoid trapping at channel walls.

In addition, in some aspects, at least some of the inlet channels 408 or outlet channels 410, or both, may also be tapered transversally over at least a portion of the longitudinal dimension forming the active contact area 422. By way of example, a channel depth may begin at 0.5 mm and taper to a point. As described, such configurations may be advantageous for controlling a pressure differential profile in the channels in order to regulate perfusion through the membrane 406 in the active contact area 422.

Referring particularly to the cross-sectional view of FIG. 4C, an inlet channels 408 and outlet channels 410 are shown to be tapered transversally, forming a taper angle α with the longitudinal direction x. That is, a surface 424 of the inlet channels 408, or outlet channel 410, or both, forms the taper angle with the longitudinal direction, or x direction, as shown. By way of example, the taper angle α can have values between 0 and 5 degrees, although other taper angles values may be possible. In some aspects, the taper angle associated with inlet channels 408 and outlet channels 410 need not be the same. As a result of the taper in the channels, fluid media introduced via the inlet 418 and extracted via the outlet 420 would experience a uniform differential pressure profile across the active contact area 422, as indicated by the arrows. Herein, the pressure differential profile refers to the various pressure differences between the inlet channels 408 and outlet channels 410 present different points along the longitudinal direction x .

By way of example, FIG. 5A-B show one embodiment of the bioreactor system described with reference to FIG. 2. Specifically referring to FIG. 5A, the system 500 may include a syringe pump 502 fluidly connected, using plastic tubing 504, to a bioreactor chip assembly 506, as described with reference to FIGs. 4A-4C. In particular, the tubing 504 may be fitted using Luer Lock connectors 508 in order to provide detachable, leak-proof connections to the syringe pump 502 as well as to an external output. As shown, the bioreactor chip assembly 506 includes a bioreactor 550 fastened to a standard microplate-sized base 510 using a chip clamps 512. Referring particularly to FIG. 5B, the bioreactor 550 is shown to include a plurality of channels 552, or chambers, formed in PDMS substrates included therein, and connected to respective inlet 554 and outlet 556 ports. As described, inlet and outlet channels 552 are separated by a permeable membrane 558. The bioreactor 550 also includes glass slides 560, with or without holes, arranged on the top and bottom of the PDMS substrates. The bioreactor 550 further includes a PDMS molded track 562 for achieving a hermetic seal.

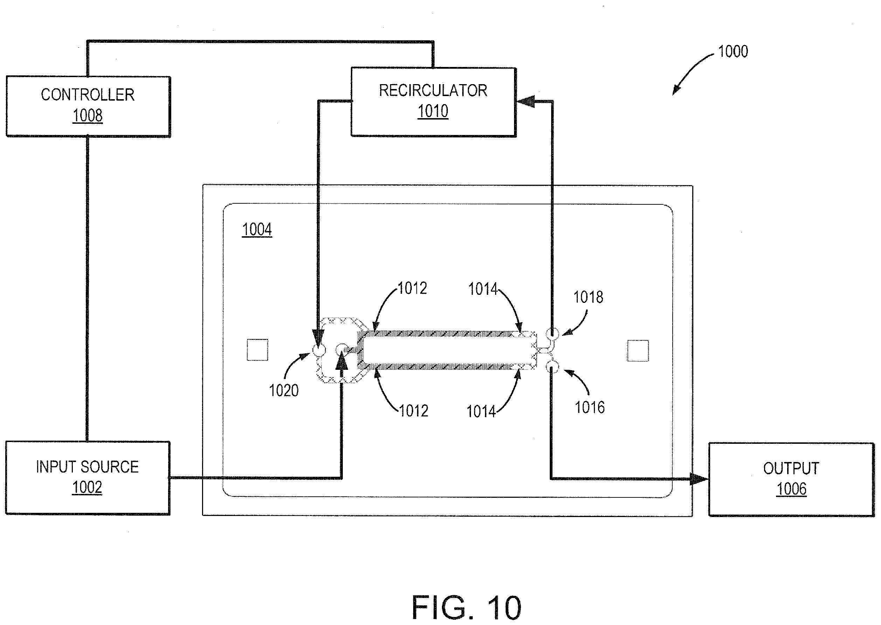

Another embodiment the bioreactor system described with reference to FIG. 2 is shown in FIG. 10. Specifically, the system 1000 includes a biological input source 1002, a bioreactor 1004, an output 1006, a controller 1008, and a recirculator 1010. As described, the input source 1002 is fluidly connected to a plurality of inlet channels 1012, while the output 1006 is fluidly connected to a plurality of outlet channels 1014, with the inlet and outlet channels being separated by a permeable membrane (not shown in FIG. 10). As described, effluent containing produced biological products, such as platelets, may be received by the output 1006.

In addition to the outlet port 1016 configured to direct effluent from the outlet channels 1014 to the output 1006 for collection, storage or further processing, the bioreactor 1004 shown in FIG. 10 also includes another outlet port 1018 connected to the outlet channels 1014. Such configuration allows effluent flowing out of the outlet channels 1014 to be redirected, using the recirculator 1010, back into the outlet channels 1014 by way of an inlet port 1020 connected to the outlet channels 1014, as indicated by arrows in FIG. 10. This allows for reduced operational volumes as well as the ability to concentrate produced biological products. In addition, pressure differentials and shear stress profiles in the channels can be independently controlled by the controller 1008. By way of example, the recirculator 1010 may be a peristaltic pump, which may be configured to achieve physiologically relevant conditions.

Although not shown, the bioreactor 1004 may also include a perfusion channel for perfusing gas, such as CO₂, into the channels. In addition, the bioreactor 1004 may be included in a bioreactor assembly capable of assembly and disassembly.

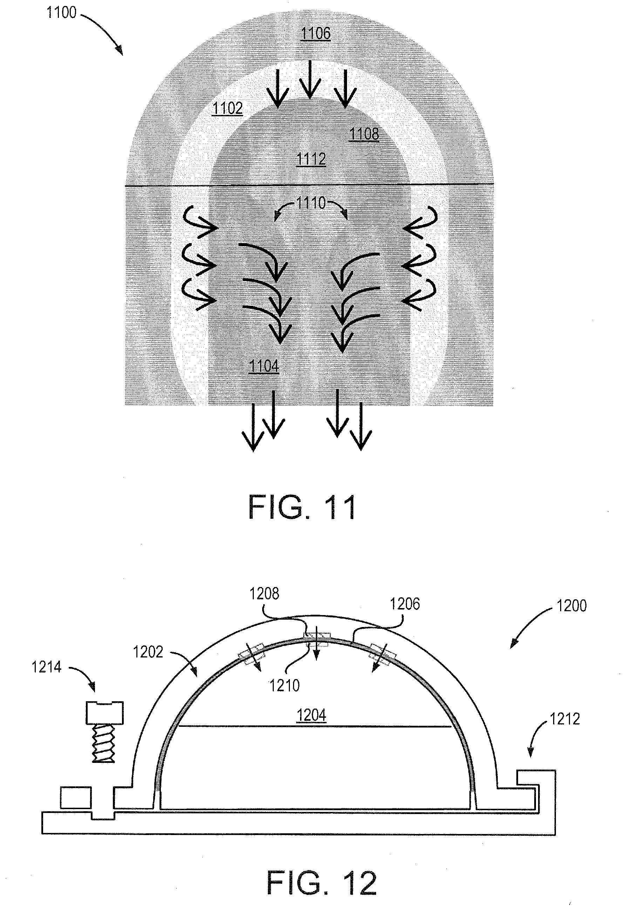

Embodiments of bioreactor systems described thus far need not be limited to planar geometries. For example, as shown in FIGs. 11 and 12, cylindrical geometries may also be implemented. Specifically referring to FIG. 11, a cross-section of an example cylindrical bioreactor 1100 is shown, which includes an outer chamber 1102 and inner chamber 1104 formed in a substrate 1106. As shown, the chambers are separated a porous membrane 1108, which is configured to form microfluidic pathways connecting the chambers, as described. The chambers are each connected to various inlet and outlet ports (not shown in FIG. 11) that may facilitate infusion and collection of fluid media flowing therethrough, as indicated by the arrows. In some aspects, as shown in FIG. 11, an inner surface 1110 of the substrate 1112 includes a curvature, or continuous taper, configured to control fluid flow between the chambers. In some aspects, the curvature may be configured to achieve a uniform pressure differential across an active area of the permeable membrane 1108.

In another example, FIG. 12 shows a cross-section of a cylindrical bioreactor assembly 1200. In general, the bioreactor assembly 1200 includes a first substrate 1202 separated from a second substrate 1204 by a permeable membrane 1206. As may be appreciated, this configuration is similar to the one described with reference to FIG. 4B, but adapted to a cylindrical geometry. Specifically, the first substrate 1202 includes a plurality of inlet channels 1208 formed therein. Similarly, the second substrate 1204 includes a plurality of outlet channels 1210 formed therein, wherein the inlet channels 1208 and outlet channels 1210 extend substantially along a longitudinal direction perpendicular to the cross-section shown in FIG. 12, and overlap over an active contact area. The channels may or may not be tapered. A fluid medium flowing in the inlet channels 1208 would then traverse the permeable membrane 1206 radially into the outlet channels 1210, as indicated by the arrows.

The channels of the bioreactor assembly 1200 may be connected to various inlet/outlet ports, and inlet/outlet manifolds (not shown in FIG. 12), similar to configurations described with reference to FIG. 3. In addition, in some aspects, the channels may be tapered transversally over at least a portion of the longitudinal direction in order to control a pressure differential regulating perfusion through the permeable membrane 1206. When the substrates are brought into engagement, by way of a base plate 1212 and fastener 1214, an even pressure is applied across the permeable membrane 1206, forming a hermetic seal between the substrates and permeable membrane 1206.

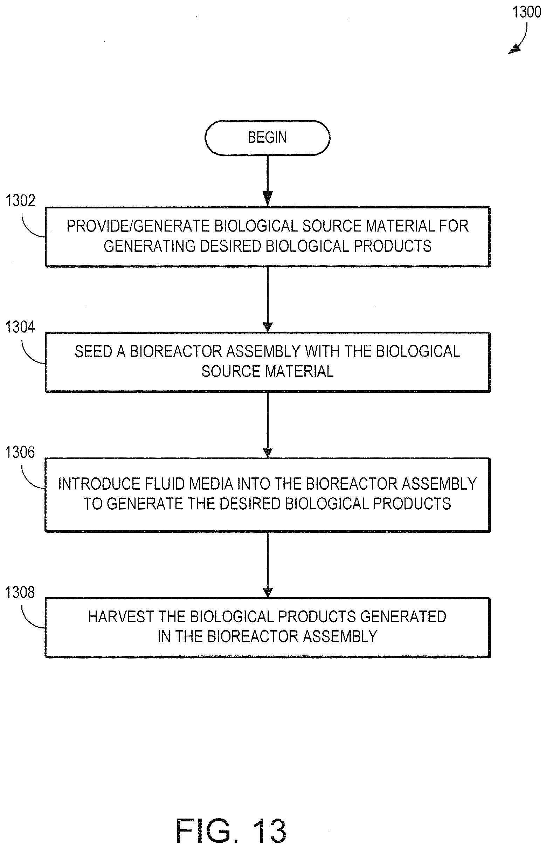

Turning now to FIG. 13, a flowchart setting forth steps of a process 1300, in accordance with aspects of the present disclosure, is shown. The process 1300 may begin at process block 1302 with providing biological source material, such as megakaryocytes or progenitor cells or stem cells. In some aspects, biological source material may also be generated at process block 1302. For instance, megakaryocytes may be generated by first isolating stem cells from umbilical cord blood, for example, and expanding them using various reagents. Such expanded cells may then be differentiated into megakaryocytic lineage, followed by a step of inducing polyploidization to generate mature megakaryocytes usable producing platelets. Alternatively, megakaryocytes may be obtained from induced pluripotent cells.

As indicated by process block 1304, the provided or generated biological source material may then be seeded in a bioreactor assembly, for instance, as described with reference to FIGs. 3-5. In some aspects, this step may include preparing various components bioreactor assembly. For example, a permeable membrane may be seeded with biological source material using various culture and plating techniques prior to device assembly. Alternatively, or additionally, a number of infusion, incubation, and other steps may also be performed to prepare the bioreactor assembly with the biological source material. For instance, megakaryocytes dispersed in a fluid medium may be selectively infused in the bioreactor assembly. By virtue of appropriately sized pores or microchannels, megakaryocytes may then be captured in a permeable membrane.

In some aspects, as described, the bioreactor assembly may be functionalized with various biological substances and compositions to optimize the production of desired biological product. For example, physiological conditions found in bone marrow and blood vessels may be reproduced to replicate *in vivo* platelet production. This may be achieved by selective infusion, or other preparation, as described steps. For instance, the bioreactor assembly may be functionalized with various cells including endothelial cells, bone marrow cells, blood cells, and cells comprising basement membranes. The bioreactor assembly may also be functionalized with various small molecules including CCL5, CXCL12, CXCL10, SDF-1, FGF-4, S1PR1, RGDS, Methylcellulose, and extracellular matrix proteins, including collagen, fibrinectin, fibrinogen, laminin, vitronectin, and combinations thereof. Such selective infusion of various biological compositions may be achieved sequentially or in parallel. In some aspects, parallel infusion may be performed, using multiple inlets and outlets, such that laminar flow media streams do not mix. Any of above biological substances or compositions may be infused using various fluid media, including cell culture media, whole blood, plasma, platelet additive solutions, suspension media, and so on. In some aspects, the above infusion and seeding processes may be visually monitored using a camera, a microscope, and the like, to verify adequate conditioning and coverage. In addition, various conditions, including temperature, light or vibration may be adjusted during performing either process block 1302 or process block 1304.

Referring again to FIG. 13, at process block 1306 fluid media may then be introduced into a seeded and functionalized bioreactor assembly in order to produce desired biological products, such as platelets. By controlling fluid media flow rates and pathways in a selected bioreactor assembly embodiment, as described, conditions can controlled to facilitate or optimize production of desired biological products. In particular, perfusion rates through the permeable membrane, along with shear stresses due to the traversing fluid can be controlled. By way of example, flow rates may be in a predetermined range approximately between 5,000 and 150,000 microliters per hour, although other values are possible, depending upon the application, specific bioreactor assembly embodiment, and desired biological products. For instance, flow rates may vary depending whether the bioreactor assembly is being prepared, or being operated to generate desired biological products.

In some aspects, flow rates may be configured to maintain shear rates in a predetermined range advantageous for efficient production of desired biological products, such as platelets. In general, such predetermined range may be between 10 s⁻¹ and 10,000 s⁻¹, although other values may be possible. In some aspects, physiological shear rates consistent with proplatelet extension and platelet production *in vivo* may be desirable. For example, physiological shear rates may be between 500 s⁻¹ and 2500 s⁻¹.

Then, at process block 1308, biological products generated in the bioreactor assembly may then be harvested. For instance, generated biological products carried by traversing fluid media and may be collected and separated from the effluent for subsequent use. In some aspects, post-collection processing may be performed. For instance, process block 1308 may also include a process to dialyze the bioreactor-derived platelets in an FDA-approved storage media, such as platelet additive solution. In particular, a dynamic dialysis system may be used, for instance, using continuous flow at low shear through a 0.75mm, 0.65µ mPES lumen (Spectrum Labs). Thus, the culture media may be replaced with a media that can be infused into human patients. In addition, in some aspects, the post-collection processing at process block 1308 may also include a process to irradiate the biological products generated. Such step is often required by the FDA before platelets can be used on human patients.

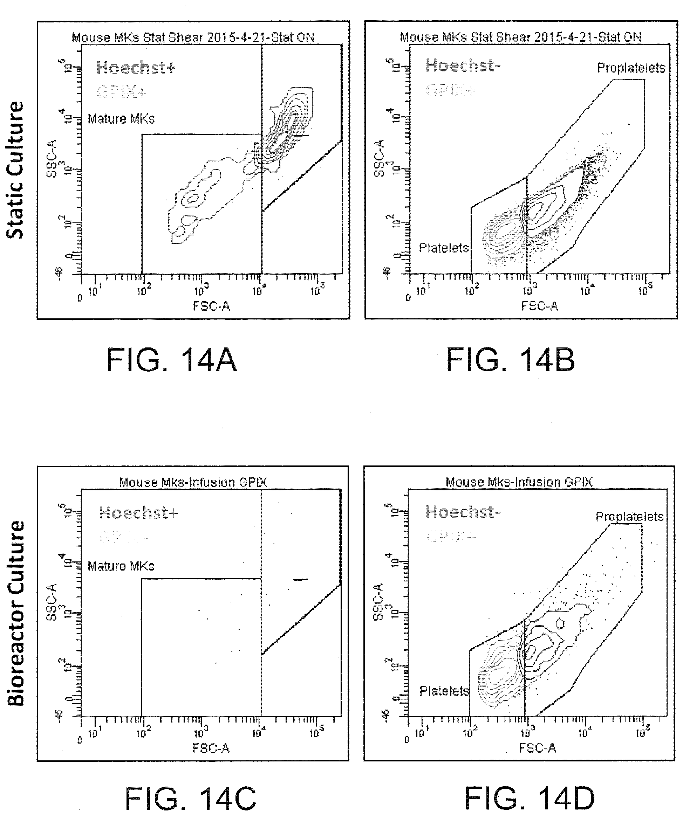

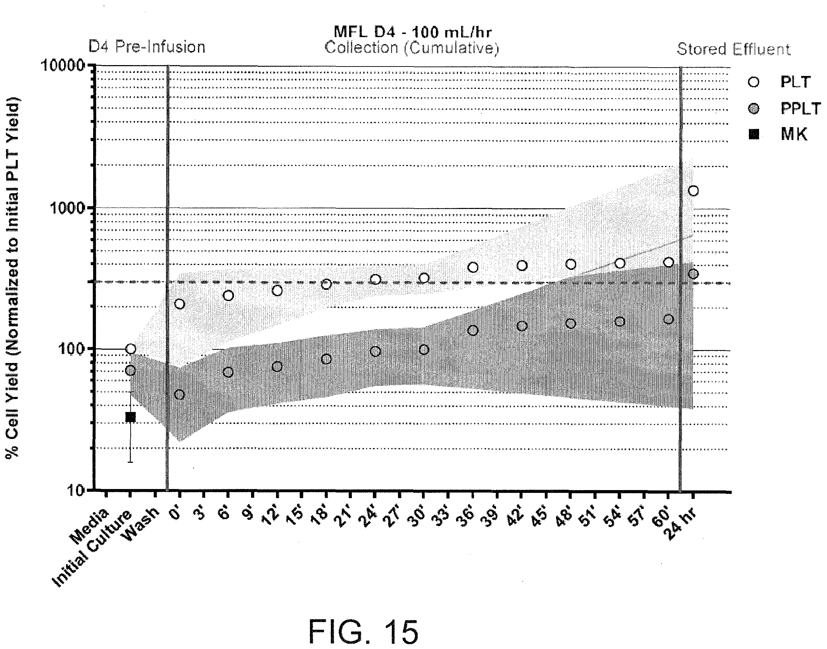

In summary, the present disclosure provides a novel approach for efficient and scalable production of platelets, and other biological products. By way of example, FIG. 14A-D show flow cytometry graphs comparing animal results of mature megakaryocyte, and proplatelet/platelet production using previous static culture techniques with those obtained using the approach of the present disclosure. In particular, FIG. 14A and 14B show mature megakaryocytes and proplatelets/platelets produced in static cultures, respectively, while FIG. 14C and 14D show mature megakaryocytes and proplatelets/platelets successfully produced using the present approach, respectively. In addition, FIG. 14C illustrates successful confinement of megakaryocytes. As another example, FIG. 15 shows a graph illustrating a timeline of cell yield using a bioreactor and method, as described. As shown, a significant number of platelets ("PLT") can be generated within the first hour of operation of the bioreactor. These results indicate that the present approach can be successfully implemented to produce clinically-relevant numbers of platelets.

The various configurations presented above are merely examples and are in no way meant to limit the scope of this disclosure. Variations of the configurations described herein will be apparent to persons of ordinary skill in the art, such variations being within the intended scope of the present application. In particular, features from one or more of the above-described configurations may be selected to create alternative configurations comprised of a sub-combination of features that may not be explicitly described above. In addition, features from one or more of the above-described configurations may be selected and combined to create alternative configurations comprised of a combination of features which may not be explicitly described above. Features suitable for such combinations and sub-combinations would be readily apparent to persons skilled in the art upon review of the present application as a whole. The subject matter described herein and in the recited claims intends to cover and embrace all suitable changes in technology.

## Claims

1. A system (100) for generating biological products, the system comprising:
a first substrate (302) having formed therein a number of inlet channels (308) extending substantially along a longitudinal direction;
a second substrate (306) having formed therein a number of outlet channels (310) corresponding to the number of inlet channels (308) and extending substantially along the longitudinal direction, the second substrate (306) configured to releasably engage the first substrate (302); and
arranged between adjacent surfaces of the substrates (302, 306), a permeable membrane (304) forming microfluidic pathways between the respective inlet and outlet channels (308, 314) and configured to selectively capture biological source material capable of generating biological products,
wherein in a cross-sectional view of the system that includes the longitudinal direction and a height direction at least one of a given inlet channel or a given outlet channel corresponding to the given inlet channel is tapered relative to the longitudinal direction at a first angle to control a pressure differential profile regulating perfusion of a fluid medium through the permeable membrane (304), and
wherein the tapering of the at least one of the given inlet channel or the given outlet channel relative to the longitudinal direction adjusts a shear stress in the channels.

2. The system (100) of claim 1, wherein the substrates (302, 206), when engaged, are configured to form a hermetic seal between respective inlet and outlet channels.

3. The system (100) of claim 1, wherein the first angle is in a range approximately between 0 and 5 degrees.

4. The system (100) of claim 1, wherein the first substrate includes a plurality of inlet channels extending along the longitudinal direction,
wherein the second substrate includes a plurality of outlet channels corresponding to the plurality of inlet channels and extending along the longitudinal direction, and
wherein the permeable membrane forms microfluidic pathways between the respective inlet and outlet channels, and
wherein the plurality of inlet channels (308) are connected to an inlet manifold (312) formed in the first substrate (302), the inlet manifold (312) being configured to uniformly or differentially distribute across the plurality of inlet channels (308) the biological source material introduced therein.

5. The system (100) of claim 4, wherein the system (100) further comprises an outlet manifold (316) formed in the second substrate (306), the outlet manifold (316) connected to the outlet channels (314) and configured to at least direct the fluid medium comprising generated biological products to an output.

6. The system (100) of claim 4, wherein the biological source material includes megakaryocytes and the biological products include platelets or megakaryocyte component products.

7. The system (100) of claim 1, wherein the system (100) further comprises a source (102) configured to selectively introduce into the channels, a fluid media comprising one or more biological substances.

8. The system (100) of claim 7, wherein the source (102) is further configured to selectively introduce fluid media comprising cells including megakaryocytes, endothelial cells, bone marrow cells, blood cells, lung cells and cells comprising basement membranes, small molecules including CCL5, CXCL12, CXCL10, SDF-1, FGF-4, S1PR1, RGDS, Methylcellulose, and extracellular matrix proteins, including collagen, fibrinectin, fibrinogen, laminin, vitronectin, and combinations thereof.

9. The system (100) of claim 7, wherein the source (102) is further configured to selectively introduce cell culture media, whole blood, plasma, platelet additive solutions, suspension media, and combinations thereof.

10. The system (100) of claim 7, wherein the source (102) is further configured to control flow of the fluid medium in the channels to generate shear rates within a predetermined range that is selected to facilitate production of the biological products.

11. The system (100) of claim 10, wherein the source (102) is further configured to control the flow to generate physiological shear rates in a range approximately between 10 sec-1 and 2000 sec-1.

12. The system (100) of claim 1, wherein the substrates comprise PDMS, thermoplastics, zeonor cyclo olefin polymers, glass, and combinations thereof.

13. The system (100) of claim 1, wherein the permeable membrane (304) comprises PDMS, thermoplastics, silk, hydrogels, or polycarbonate.

14. The system (100) of claim 1, wherein the permeable membrane (304) comprises pores sized in a range approximately between 3 micrometers and 10 micrometers.

15. The system (100) of claim 1, wherein
the first angle is defined between a first surface of the given inlet channel and the longitudinal direction,
wherein in a cross-sectional view of the system that includes the longitudinal direction and a height direction the given outlet channel is tapered relative to the longitudinal direction at a second angle, the second angle defined between a second surface of the given outlet channel and the longitudinal direction,
wherein the first angle is negative and the second angle is positive.

16. The system of claim 15, wherein a magnitude of the first angle and a magnitude of the second angle are identical.

17. The system (100) of claim 1, wherein the at least one of the given inlet channel or the given outlet channel corresponding to the given inlet channel is tapered relative to the longitudinal direction at the first angle such that the depth of at least one of the given inlet channel or the given outlet channel changes along the longitudinal direction to control the pressure differential profile regulating perfusion of the fluid medium through the permeable membrane, such that an active area defined by an overlap of the given inlet channel and the given outlet channel that includes a portion of the permeable membrane has a substantially uniform pressure differential profile along the longitudinal direction.

## Patentansprüche

1. System (100) zur Erzeugung biologischer Produkte, wobei das System umfasst:
ein erstes Substrat (302), in dem eine Anzahl von Einlasskanälen (308) ausgebildet ist, die sich im Wesentlichen entlang einer Längsrichtung erstrecken;
ein zweites Substrat (306), in dem eine Anzahl von Auslasskanälen (310) ausgebildet ist, die der Anzahl von Einlasskanälen (308) entspricht und sich im Wesentlichen entlang der Längsrichtung erstreckt, wobei das zweite Substrat (306) so konfiguriert ist, dass es lösbar mit dem ersten Substrat (302) in Eingriff steht; und
eine zwischen benachbarten Oberflächen der Substrate (302, 306) angeordnete durchlässige Membran (304), die mikrofluidische Pfade zwischen den jeweiligen Einlass- und Auslasskanälen (308, 314) bildet und so konfiguriert ist, dass sie biologisches Ausgangsmaterial, das biologische Produkte erzeugen kann, selektiv einfängt,
wobei in einer Querschnittsansicht des Systems, die die Längsrichtung und eine Höhenrichtung einschließt, mindestens einer von einem gegebenen Einlasskanal oder einem gegebenen Auslasskanal, der dem gegebenen Einlasskanal entspricht, relativ zu der Längsrichtung in einem ersten Winkel verjüngt ist, um ein Druckdifferenzprofil zu steuern, das die Perfusion eines fluiden Mediums durch die durchlässige Membran (304) reguliert, und
wobei die Verjüngung des gegebenen Einlasskanals und/oder des gegebenen Auslasskanals in Bezug auf die Längsrichtung eine Scherspannung in den Kanälen einstellt.

2. System (100) nach Anspruch 1, wobei die Substrate (302, 206), wenn sie ineinandergreifen, so konfiguriert sind, dass sie eine hermetische Abdichtung zwischen den jeweiligen Einlass- und Auslasskanälen bilden.

3. System (100) nach Anspruch 1, wobei der erste Winkel in einem Bereich von ungefähr zwischen 0 und 5 Grad liegt.

4. System (100) nach Anspruch 1, wobei das erste Substrat eine Vielzahl von Einlasskanälen aufweist, die sich entlang der Längsrichtung erstrecken,
wobei das zweite Substrat eine Vielzahl von Auslasskanälen umfasst, die der Vielzahl von Einlasskanälen entsprechen und sich entlang der Längsrichtung erstrecken, und
wobei die permeable Membran mikrofluidische Pfade zwischen den jeweiligen Einlass- und Auslasskanälen bildet, und
wobei die mehreren Einlasskanäle (308) mit einem Einlassverteiler (312) verbunden sind, der in dem ersten Substrat (302) ausgebildet ist, wobei der Einlassverteiler (312) so konfiguriert ist, dass er das in die mehreren Einlasskanäle (308) eingebrachte biologische Ausgangsmaterial gleichmäßig oder differenziert über diese verteilt.

5. System (100) nach Anspruch 4, wobei das System (100) ferner einen Auslassverteiler (316) umfasst, der in dem zweiten Substrat (306) ausgebildet ist, wobei der Auslassverteiler (316) mit den Auslasskanälen (314) verbunden und so konfiguriert ist, dass er zumindest das flüssige Medium, das erzeugte biologische Produkte umfasst, zu einem Ausgang leitet.

6. System (100) nach Anspruch 4, wobei das biologische Ausgangsmaterial Megakaryozyten umfasst und die biologischen Produkte Blutplättchen oder Megakaryozytenkomponentenprodukte umfassen.

7. System (100) nach Anspruch 1, wobei das System (100) ferner eine Quelle (102) umfasst, die so konfiguriert ist, dass sie selektiv ein flüssiges Medium in die Kanäle einführt, das eine oder mehrere biologische Substanzen umfasst.

8. System (100) nach Anspruch 7, wobei die Quelle (102) ferner dazu eingerichtet ist, dass sie selektiv flüssige Medien einführt, die Zellen umfassen, einschließlich Megakaryozyten, Endothelzellen, Knochenmarkzellen, Blutzellen, Lungenzellen und Zellen, die Basalmembranen umfassen, kleine Moleküle, einschließlich CCL5, CXCL12, CXCL10, SDF-1, FGF-4, S1PR1, RGDS, Methylcellulose, und extrazelluläre Matrixproteine, einschließlich Kollagen, Fibrinectin, Fibrinogen, Laminin, Vitronectin und Kombinationen davon.

9. System (100) nach Anspruch 7, wobei die Quelle (102) ferner so konfiguriert ist, dass sie selektiv Zellkulturmedien, Vollblut, Plasma, Thrombozytenzusatzlösungen, Suspensionsmedien und Kombinationen davon einführt.

10. System (100) nach Anspruch 7, wobei die Quelle (102) ferner dazu eingerichtet ist, dass sie die Strömung des flüssigen Mediums in den Kanälen steuert, um Scherraten innerhalb eines vorbestimmten Bereichs zu erzeugen, der so gewählt ist, dass die Herstellung der biologischen Produkte erleichtert wird.

11. System (100) nach Anspruch 10, wobei die Quelle (102) ferner dazu eingerichtet ist, dass sie die Strömung so steuert, dass physiologische Schergeschwindigkeiten in einem Bereich von etwa zwischen 10 s-1 und 2000 s-1 erzeugt werden.

12. System (100) nach Anspruch 1, wobei die Substrate PDMS, Thermoplaste, Zeonor-Cyclo-Olefin-Polymere, Glas und Kombinationen davon umfassen.

13. System (100) nach Anspruch 1, wobei die durchlässige Membran (304) PDMS, Thermoplaste, Seide, Hydrogele oder Polycarbonat umfasst.

14. System (100) nach Anspruch 1, wobei die durchlässige Membran (304) Poren mit einer Größe in einem Bereich von ungefähr zwischen 3 Mikrometern und 10 Mikrometern aufweist.

15. System (100) nach Anspruch 1, wobei
der erste Winkel zwischen einer ersten Oberfläche des gegebenen Einlasskanals und der Längsrichtung definiert ist,
wobei in einer Querschnittsansicht des Systems, die die Längsrichtung und eine Höhenrichtung einschließt, der gegebene Auslasskanal relativ zur Längsrichtung unter einem zweiten Winkel verjüngt ist, wobei der zweite Winkel zwischen einer zweiten Oberfläche des gegebenen Auslasskanals und der Längsrichtung definiert ist,
wobei der erste Winkel negativ ist und der zweite Winkel positiv ist.

16. System nach Anspruch 15, wobei ein Größenwert des ersten Winkels und ein Größenwert des zweiten Winkels identisch sind.

17. System (100) nach Anspruch 1, wobei der gegebene Einlasskanal und/oder der gegebene Auslasskanal, der dem gegebenen Einlasskanal entspricht, relativ zur Längsrichtung unter dem ersten Winkel verjüngt ist, so dass sich die Tiefe des gegebenen Einlasskanals und/oder des gegebenen Auslasskanals entlang der Längsrichtung ändert, um das Druckdifferenzprofil zu steuern, das die Perfusion des fluiden Mediums durch die durchlässige Membran reguliert, so dass ein aktiver Bereich, der durch eine Überlappung des gegebenen Einlasskanals und des gegebenen Auslasskanals definiert ist, der einen Teil der durchlässigen Membran umfasst, ein im Wesentlichen einheitliches Druckdifferenzprofil entlang der Längsrichtung aufweist.

## Revendications

1. Système (100) pour la production de produits biologiques, le système comprenant :
un premier substrat (302) dans lequel est formé un certain nombre de canaux d'entrée (308) s'étendant sensiblement le long d'une direction longitudinale ;
un deuxième substrat (306) dans lequel est formé un nombre de canaux de sortie (310) correspondant au nombre de canaux d'entrée (308) et s'étendant sensiblement le long de la direction longitudinale, le deuxième substrat (306) étant configuré pour s'engager de manière amovible avec le premier substrat (302) ; et agencée entre les surfaces adjacentes des substrats (302, 306), une membrane perméable (304) formant des voies microfluidiques entre les canaux d'entrée et de sortie respectifs (308, 314) et configurée pour capturer sélectivement un matériau source biologique capable de générer des produits biologiques,
dans lequel, dans une vue en coupe transversale du système qui comprend la direction longitudinale et une direction de hauteur, au moins l'un d'un canal d'entrée donné ou d'un canal de sortie donné correspondant au canal d'entrée donné est effilé par rapport à la direction longitudinale selon un premier angle pour contrôler un profil de pression différentielle régulant la perfusion d'un milieu fluide à travers la membrane perméable (304), et
dans lequel l'effilement d'au moins l'un du canal d'entrée donné ou du canal de sortie donné par rapport à la direction longitudinale ajuste une contrainte de cisaillement dans les canaux.

2. Système (100) de la revendication 1, dans lequel les substrats (302, 206), lorsqu'ils sont engagés, sont configurés pour former un joint hermétique entre les canaux d'entrée et de sortie respectifs.

3. Système (100) de la revendication 1, dans lequel le premier angle est dans une plage approximativement comprise entre 0 et 5 degrés.

4. Système (100) de la revendication 1, dans lequel le premier substrat comprend une pluralité de canaux d'entrée s'étendant le long de la direction longitudinale,
dans lequel le second substrat comprend une pluralité de canaux de sortie correspondant à la pluralité de canaux d'entrée et s'étendant le long de la direction longitudinale, et
dans lequel la membrane perméable forme des voies microfluidiques entre les canaux d'entrée et de sortie respectifs, et
dans lequel la pluralité de canaux d'entrée (308) est connectée à un collecteur d'entrée (312) formé dans le premier substrat (302), le collecteur d'entrée (312) étant configuré pour distribuer uniformément ou différentiellement à travers la pluralité de canaux d'entrée (308) le matériau de source biologique introduit dans celui-ci.

5. Système (100) de la revendication 4, dans lequel le système (100) comprend en outre un collecteur de sortie (316) formé dans le second substrat (306), le collecteur de sortie (316) étant connecté aux canaux de sortie (314) et configuré pour au moins diriger le milieu fluide comprenant les produits biologiques générés vers une sortie.

6. Système (100) de la revendication 4, dans lequel le matériau source biologique comprend des mégacaryocytes et les produits biologiques comprennent des plaquettes ou des produits de composants mégacaryocytaires.

7. Système (100) de la revendication 1, dans lequel le système (100) comprend en outre une source (102) configurée pour introduire sélectivement dans les canaux, un milieu fluide comprenant une ou plusieurs substances biologiques.

8. Système (100) de la revendication 7, dans lequel la source (102) est en outre configurée pour introduire sélectivement dans les canaux un milieu fluide comprenant des cellules incluant des mégacaryocytes, des cellules endothéliales, des cellules de moelle osseuse, des cellules sanguines, des cellules pulmonaires et des cellules comprenant des membranes basales, des petites molécules comprenant CCL5, CXCL12, CXCL10, SDF-1, FGF-4, S1PR1, RGDS, Methylcellulose, et des protéines de la matrice extracellulaire, comprenant le collagène, la fibrinectine, le fibrinogène, la laminine, la vitronectine, et des combinaisons de ceux-ci.

9. Système (100) de la revendication 7, dans lequel la source (102) est en outre configurée pour introduire sélectivement des milieux de culture cellulaire, du sang total, du plasma, des solutions d'additifs plaquettaires, des milieux de suspension, et des combinaisons de ceux-ci.

10. Système (100) de la revendication 7, dans lequel la source (102) est en outre configurée pour contrôler l'écoulement du milieu fluide dans les canaux pour générer des taux de cisaillement dans une plage prédéterminée qui est sélectionnée pour faciliter la production des produits biologiques.

11. Système (100) selon la revendication 10, dans lequel la source (102) est en outre configurée pour contrôler l'écoulement pour générer des taux de cisaillement physiologiques dans une plage approximativement comprise entre 10 sec-1 et 2000 sec-1.

12. Système (100) de la revendication 1, dans lequel les substrats comprennent du PDMS, des thermoplastiques, des polymères de cyclo-oléfine zeonor, du verre, et des combinaisons de ceux-ci.

13. Système (100) de la revendication 1, dans lequel la membrane perméable (304) comprend du PDMS, des thermoplastiques, de la soie, des hydrogels ou du polycarbonate.

14. Système (100) de la revendication 1, dans lequel la membrane perméable (304) comprend des pores dimensionnés dans une plage approximativement comprise entre 3 micromètres et 10 micromètres.

15. Système (100) de la revendication 1, dans lequel
le premier angle est défini entre une première surface du canal d'entrée donné et la direction longitudinale,
dans lequel, dans une vue en coupe transversale du système qui comprend la direction longitudinale et une direction de hauteur, le canal de sortie donné est effilé par rapport à la direction longitudinale selon un second angle, le second angle étant défini entre une seconde surface du canal de sortie donné et la direction longitudinale,
dans lequel le premier angle est négatif et le second angle est positif.

16. Système de la revendication 15, dans lequel une magnitude du premier angle et une magnitude du second angle sont identiques.

17. Système (100) de la revendication 1, dans lequel le au moins un du canal d'entrée donné ou du canal de sortie donné correspondant au canal d'entrée donné est effilé par rapport à la direction longitudinale au niveau du premier angle de sorte que la profondeur d'au moins un du canal d'entrée donné ou du canal de sortie donné change le long de la direction longitudinale pour contrôler le profil de pression différentielle régulant la perfusion du milieu fluide à travers la membrane perméable, de sorte qu'une zone active définie par un chevauchement du canal d'entrée donné et du canal de sortie donné qui comprend une partie de la membrane perméable a un profil de pression différentielle sensiblement uniforme le long de la direction longitudinale.
